# EUROPEAN PATENT APPLICATION

(11) **EP 4 616 868 A1**
(43) Date of publication of application: **17.09.2025**
(21) Application number: 23888052.0
(22) Date of filing: 08.11.2023
(51) Int. Cl.: A61K 45/06, A61K 39/395, A61K 31/435, A61K 31/415, A61K 31/495, A61P 35/00

(54) **PHARMACEUTICAL COMPOSITION OF RIP2 INHIBITOR IN COMBINATION WITH IMMUNE CHECKPOINT INHIBITOR AND USE THEREOF**

(30) Priority: 09.11.2022 CN 202211400189
(71) Applicant: Ningbo Combireg Pharmaceutical Technology Co., Ltd, Ningbo City, Zhejiang Province, 315336 (CN)
(72) Inventor: LIU, Gang, Hangzhou Bay New Zone Ningbo, Zhejiang 315336 (CN); MA, Yao, Hangzhou Bay New Zone Ningbo, Zhejiang 315336 (CN); WEI, Xiduan, Hangzhou Bay New Zone Ningbo, Zhejiang 315336 (CN)
(74) Representative: Murgitroyd & Company
(86) International application number: PCT/CN2023/130489
(87) International publication number: WO 2024/099363

(57) **Abstract**

Disclosed are a pharmaceutical composition of a receptor-interacting protein 2 (RIP2) inhibitor in combination with an immune checkpoint inhibitor and use thereof. The present invention provides a pharmaceutical composition comprising an RIP2 inhibitor and an immune checkpoint inhibitor (ICI). Particularly, the present invention provides use of the RIP2 inhibitor in combination with the ICI in the preparation of a medicament for treating and/or preventing tumors. The knockout of the RIP2 or the RIP2 inhibitor has a new application of increasing the sensitivity of tumors to the ICI, i.e., has the effect of enhancing the anti-tumor activity of an ICI medicament. According to the present invention, the anti-tumor activity of an ICI medicament can be enhanced by knocking out the RIP2 or using the RIP2 inhibitor for the first time. After the RIP2 inhibitor and the ICI are jointly used, the proportions of CD3+ cells, CD8+ T cells, γδTCR+ T cells, effector memory T cells, and CD8 effector T cells (IFNγ+ CD8+ T cells, Granzyme B+ CD8+ T cells, TNFα+ CD8+ T cells, and CD107a+ CD8+ T cells) in a tumor microenvironment are up-regulated, and the therapeutic effect of the ICI medicament on tumors is enhanced.

## Description

The present application claims priority to Chinese patent application 202211400189.4 filed on November 9, 2022, the contents of which are incorporated herein by reference in their entirety.

### TECHNICAL FIELD

The present disclosure belongs to the technical field of tumor therapy and relates to a pharmaceutical composition comprising a receptor-interacting protein 2 (RIP2) inhibitor in combination with an immune checkpoint inhibitor (ICI) and a use thereof. Specifically, the present disclosure relates to a use of RIP2 inhibitors in enhancing the efficacy of anti-tumor ICI medicaments.

### BACKGROUND

Tumors are one of the major threats to human health, and the mortality rate from tumors has shown an increasing trend globally year by year. Traditional tumor treatments include surgery, radiotherapy, chemotherapy, and targeted drug therapy. In recent years, with in-depth understanding and research of the tumor immune system, tumor immunotherapy has been widely used in clinical practice and has continuously achieved breakthroughs in efficacy. Tumor immunotherapy works by reactivating suppressed specific immune responses and restoring the normal anti-tumor immune activity of the body, thereby controlling and eliminating tumors. Immune checkpoint therapy is an important component of tumor immunotherapy, which works by regulating T cell activity through a series of pathways such as co-inhibitory or co-stimulatory signals to kill tumor cells. The immune checkpoint molecules that have been studied in depth include PD-1, PD-L1, CTLA-4, BTLA, TIM-3, TIGIT, VISTA, LAG-3, IDO1, *etc.* Immune checkpoint inhibitors (ICIs) that have been successfully used in the treatment of various tumors with good efficacy in clinical practice include PD-1 inhibitors, PD-L1 inhibitors, and CTLA-4 inhibitors. However, clinical applications have revealed significant limitations of ICIs, such as low response rates (PD-1 inhibitors exhibit response rates of only 5% to 30%, with a response rate of only 5% in patients with colorectal cancer), strong cytotoxicity, treatment resistance, and various immune-related adverse effects, among which the low response rate severely restricts the benefits of ICI therapy for tumor patients. Therefore, exploring novel therapeutic approaches to improve ICI sensitivity and increase response rates has been a longstanding research focus.

### CONTENT OF THE PRESENT INVENTION

The technical problem addressed by the present disclosure is the drawbacks such as poor effectiveness, cytotoxicity, and treatment resistance associated with ICIs. The present disclosure provides a pharmaceutical composition comprising a receptor-interacting protein 2 (RIP2) inhibitor in combination with an ICI and a use thereof. The purpose of the present disclosure is to provide a new use of knockout of RIP2 or the use of an RIP2 inhibitor to significantly sensitize tumors to ICI medicaments. In other words, the composition of the present disclosure effectively enhances the therapeutic efficacy of ICI medicaments on tumors, with high effectiveness and broad application prospects. The present disclosure is the first to discover that knockout of RIP2 or the use of an RIP2 inhibitor can enhance the anti-tumor activity of ICI medicaments. The combined use of the RIP2 inhibitor and the ICI up-regulates the proportions of CD3+ cells, CD8+ T cells, γδTCR+ T cells, effector memory T cells, and CD8 effector T cells (IFNγ+ CD8+ T cells, Granzyme B+ CD8+ T cells, TNFα+ CD8+ T cells, and CD107a+ CD8+ T cells) in a tumor microenvironment, thereby enhancing the therapeutic effect of ICI medicaments on tumors.

The present disclosure provides a pharmaceutical composition of an RIP2 inhibitor in combination with an ICI, comprising an RIP2 inhibitor and an ICI.

The pharmaceutical composition may further comprise a pharmaceutically acceptable excipient. The dosage of the pharmaceutically acceptable excipient may be conventional in the art, which may be routinely selected by those skilled in the art based on the desired dosage form, properties, size, and compatibility with the active ingredient.

The RIP2 inhibitor is preferably one or more of GSK583 (CAS No. 1346547-00-9), WEHI-345, SB203580, OD36, OD38, and a derivative thereof, and further preferably GSK583 and/or a derivative thereof.

The ICI is preferably one or more of a programmed death 1 (PD-1) inhibitor, a programmed death-ligand 1 (PD-L1) inhibitor, a cytotoxic T-lymphocyte-associated protein 4 (CTLA-4) inhibitor, a B- and T-lymphocyte attenuator (BTLA) inhibitor, a T cell immunoglobulin and mucin domain 3 (TIM-3) inhibitor, a T cell immunoreceptor with Ig and ITIM domains (TIGIT) inhibitor, a V-domain Ig suppressor of T cell activation (VISTA) inhibitor, a lymphocyte-activation gene 3 (LAG-3) inhibitor, and an indoleamine 2,3-dioxygenase 1 (IDO1) inhibitor; further preferably, the ICI comprises a PD-1 inhibitor and/or a PD-L1 inhibitor.

In the pharmaceutical composition, the programmed death 1 (PD-1) inhibitor may be pembrolizumab.

In the pharmaceutical composition, the programmed death-ligand 1 (PD-L1) inhibitor may be atezolizumab.

In the pharmaceutical composition, the derivative of the RIP2 inhibitor may be a molecule of common or uncommon types in the art, which is capable of achieving RIP2 inhibition *in vivo* through chemical and/or physical reactions, including, but not limited to, prodrugs of RIP2 inhibitors, such as ester derivatives and salt forms.

In the pharmaceutical composition, the cytotoxic T-lymphocyte-associated protein 4 (CTLA-4) inhibitor may be ipilimumab or tremelimumab.

In the pharmaceutical composition, the B- and T-lymphocyte attenuator (BTLA) inhibitor may be icatolimab.

In the pharmaceutical composition, the T cell immunoglobulin and mucin domain 3 (TIM-3) inhibitor may be cobolimab (TSR-022) or sabatolimab (MBG453).

In the pharmaceutical composition, the T cell immunoreceptor with Ig and ITIM domains (TIGIT) inhibitor may be tiragolumab.

In the pharmaceutical composition, the V-domain Ig suppressor of T cell activation (VISTA) inhibitor may be CA-170 or JNJ-610588.

In the pharmaceutical composition, the lymphocyte-activation gene 3 (LAG-3) inhibitor may be relatlimab or tebotelimab.

In the pharmaceutical composition, the indoleamine 2,3-dioxygenase 1 (IDO1) inhibitor may be epacadostat or navoximod.

In the pharmaceutical composition, the mass ratio of the RIP2 inhibitor to the ICI may be 1:(1 to 60), such as 1:6, 1:10, 1:15, 1:20, 1:30, or 1:60.

In the pharmaceutical composition, the mass ratio of the RIP2 inhibitor to the ICI may be 1:(6 to 10), such as 1:6 or 1:10.

In a certain embodiment, the pharmaceutical composition consists of the RIP2 inhibitor and the ICI.

In a certain embodiment, the RIP2 inhibitor in the pharmaceutical composition is a derivative of GSK583, and the derivative of GSK583 is preferably GSK2983559.

In a certain embodiment, the ICI in the pharmaceutical composition is a PD-1 inhibitor (*e.g.,* pembrolizumab) or a PD-L1 inhibitor (*e.g.,* atezolizumab).

In a certain embodiment, the pharmaceutical composition is GSK583 and a PD-L1 inhibitor.

In a certain embodiment, the pharmaceutical composition is GSK583 and a PD-L1 inhibitor, and the mass ratio of the GSK583 to the PD-L1 inhibitor is 1:6 to 1:10.

In a certain embodiment, the pharmaceutical composition is an RIP2 inhibitor and atezolizumab.

In a certain embodiment, the pharmaceutical composition is GSK583 and atezolizumab.

In a certain embodiment, the pharmaceutical composition is GSK2983559 and atezolizumab.

In a certain embodiment, the pharmaceutical composition is GSK583 and a PD-1 inhibitor.

In a certain embodiment, the pharmaceutical composition comprises GSK583 and a PD-1 inhibitor, and the mass ratio of the GSK583 to the PD-1 inhibitor is 1:6 to 1:10.

In a certain embodiment, the pharmaceutical composition is an RIP2 inhibitor and pembrolizumab.

In a certain embodiment, the pharmaceutical composition is GSK583 and pembrolizumab.

In a certain embodiment, the pharmaceutical composition is GSK2983559 and pembrolizumab.

In the pharmaceutical composition, the RIP2 inhibitor and the ICI may be administered simultaneously or separately.

In the pharmaceutical composition, the RIP2 inhibitor and the ICI may be administered at equal or unequal intervals.

The method of "simultaneous administration" may refer to, for example, the RIP2 inhibitor and the ICI comprised in a single pharmaceutical composition being administered simultaneously; or, a "separate pharmaceutical composition comprising the RIP2 inhibitor" and a "separate pharmaceutical composition comprising the ICI" being administered simultaneously.

The method of "separate administration" may refer to, for example, a "separate pharmaceutical composition comprising the RIP2 inhibitor" and a "separate pharmaceutical composition comprising the ICI" being administered separately at different times. For example, one of the "separate pharmaceutical composition comprising the RIP2 inhibitor" and the "separate pharmaceutical composition comprising the ICI" is administered first, followed by the other. For example, the "separate pharmaceutical composition comprising the RIP2 inhibitor" is administered first, followed by the "separate pharmaceutical composition comprising the ICI"; or, the "separate pharmaceutical composition comprising the ICI" is administered first, followed by the "separate pharmaceutical composition comprising the RIP2 inhibitor".

The "separate pharmaceutical composition comprising the RIP2 inhibitor" may be a pharmaceutical composition comprising the RIP2 inhibitor and a pharmaceutically acceptable excipient. The "separate pharmaceutical composition comprising the ICI" may be a pharmaceutical composition comprising the ICI and a pharmaceutically acceptable excipient.

The separate administration may be performed at closely spaced or widely spaced intervals. During separate administration, each separate pharmaceutical composition may be administered at equal or unequal intervals, such as once every 4 days or once every 7 days.

In the pharmaceutical composition, the RIP2 inhibitor (*e.g.,* GSK583) is administered at different doses depending on tumor characteristics (*e.g.,* tumor size, number of treatments, intervals) or body weight of a patient, typically at a dose of 0.1 mg/kg to 5 mg/kg (per single dose), preferably 1 mg/kg (per single dose).

In the pharmaceutical composition, the ICI may be administered at a dose that is conventional in the art, typically at a dose of 2 mg/kg to 10 mg/kg (per single dose), such as 6 mg/kg (per single dose). For another example, the PD-L1 inhibitor (*e.g.,* atezolizumab) is administered at a dose of 6 mg/kg (per single dose), and the PD-1 inhibitor (*e.g.,* pembrolizumab) is administered at a dose of 6 mg/kg (per single dose).

Preferably, the RIP2 inhibitor is administered at equal intervals during treatment, and the ICI is administered at equal or unequal intervals during treatment.

Preferably, the RIP2 inhibitor is administered once every 4 days.

Preferably, the ICI is administered at unequal intervals, once every 4 days or once every 7 days.

Preferably, the RIP2 inhibitor is administered once every 4 days, for a total of 4 to 7 doses, for example, on days 0, 4, 8, and 12.

Preferably, the ICI is administered at unequal intervals, once every 4 days or once every 7 days, for a total of 3, 4, or 5 doses, for example, on days 0, 4, 11, and 18.

Whether administered simultaneously or separately, the administration regimen (including route of administration, frequency of administration, dose of administration, interval of administration, *etc*.) of the RIP2 inhibitor and the ICI may be the same or different, and may be adjusted by those skilled in the art as needed to provide optimal therapeutic efficacy.

In a certain embodiment, the RIP2 inhibitor is administered via injection (*e.g.,* intravenous, subcutaneous, intraperitoneal, or intramuscular injection) or orally.

In a certain embodiment, the ICI is administered via injection (*e.g.,* intravenous, subcutaneous, intraperitoneal, or intramuscular injection).

In a certain embodiment, both the RIP2 inhibitor and the ICI are administered via injection, such as intravenous, subcutaneous, intraperitoneal, or intramuscular injection.

The present disclosure provides a use of the pharmaceutical composition in the manufacture of a medicament for treating and/or preventing a tumor.

In the use, the tumor may be a tumor related to RIP2 signaling pathway, preferably colorectal cancer, melanoma, lung cancer, breast cancer, liver cancer, gastric cancer, or pancreatic cancer.

The present disclosure provides a method for treating and/or preventing a tumor, comprising administering a therapeutically effective amount of the pharmaceutical composition to a patient in need thereof.

In the method for treating and/or preventing a tumor, the tumor may be a tumor related to RIP2 signaling pathway, preferably colorectal cancer, melanoma, lung cancer, breast cancer, liver cancer, gastric cancer, or pancreatic cancer.

In the pharmaceutical composition, the RIP2 inhibitor and the ICI may be administered at equal or unequal intervals.

In the method for treating and/or preventing a tumor, the RIP2 inhibitor and the ICI may be administered simultaneously or separately.

The method of "simultaneous administration" may refer to, for example, the RIP2 inhibitor and the ICI comprised in a single pharmaceutical composition being administered simultaneously; or, a "separate pharmaceutical composition comprising the RIP2 inhibitor" and a "separate pharmaceutical composition comprising the ICI" being administered simultaneously.

The method of "separate administration" may refer to, for example, a "separate pharmaceutical composition comprising the RIP2 inhibitor" and a "separate pharmaceutical composition comprising the ICI" being administered separately at different times. For example, one of the "separate pharmaceutical composition comprising the RIP2 inhibitor" and the "separate pharmaceutical composition comprising the ICI" is administered first, followed by the other. For another example, the "separate pharmaceutical composition comprising the RIP2 inhibitor" is administered first, followed by the "separate pharmaceutical composition comprising the ICI"; or, the "separate pharmaceutical composition comprising the ICI" is administered first, followed by the "separate pharmaceutical composition comprising the RIP2 inhibitor".

The "separate pharmaceutical composition comprising the RIP2 inhibitor" may be a pharmaceutical composition comprising the RIP2 inhibitor and a pharmaceutically acceptable excipient. The "separate pharmaceutical composition comprising the ICI" may be a pharmaceutical composition comprising the ICI and a pharmaceutically acceptable excipient.

The separate administration may be performed at closely spaced or widely spaced intervals. During separate administration, each separate pharmaceutical composition may be administered at equal or unequal intervals, such as once every 4 days or once every 7 days.

Preferably, the RIP2 inhibitor is administered at equal intervals during treatment, and the ICI is administered at equal or unequal intervals during treatment.

Preferably, the RIP2 inhibitor is administered once every 4 days, for a total of 4 to 7 doses, for example, on days 0, 4, 8, and 12.

Preferably, the ICI is administered at unequal intervals, once every 4 days or once every 7 days, for a total of 3, 4, or 5 doses, for example, on days 0, 4, 11, and 18.

In the method for treating and/or preventing a tumor, the RIP2 inhibitor may be administered at different doses depending on tumor characteristics (*e.g.,* tumor size, number of treatments, intervals) or body weight of a patient, typically at a dose of 0.1 mg/kg to 5 mg/kg (per single dose), such as 1 mg/kg (per single dose). For another example, GSK583 is administered at a dose of 1 mg/kg (per single dose).

In the method for treating and/or preventing a tumor, the ICI may be administered at different doses depending on tumor characteristics (*e.g.,* tumor size, number of treatments, intervals) or body weight of a patient, typically at a dose of 2 mg/kg to 10 mg/kg (per single dose), such as 6 mg/kg (per single dose). For another example, the PD-L1 inhibitor (*e.g.,* atezolizumab) is administered at a dose of 6 mg/kg (per single dose), and the PD-1 inhibitor (*e.g.,* pembrolizumab) is administered at a dose of 6 mg/kg (per single dose).

Whether administered simultaneously or separately, the administration regimen (including route of administration, dose of administration, interval of administration, *etc*.) of the RIP2 inhibitor and the ICI may be the same or different, and may be adjusted by those skilled in the art as needed to provide optimal therapeutic efficacy.

In a certain embodiment, the RIP2 inhibitor is administered via injection (*e.g.,* intravenous, subcutaneous, intraperitoneal, or intramuscular injection) or orally.

In a certain embodiment, the ICI is administered via injection (*e.g.,* intravenous, subcutaneous, intraperitoneal, or intramuscular injection).

In a certain embodiment, both the RIP2 inhibitor and the ICI are administered via injection, such as intravenous, subcutaneous, intraperitoneal, or intramuscular injection.

In the method for treating and/or preventing a tumor, the administration regimen (including route of administration, dose of administration, interval of administration, *etc*.) of the RIP2 inhibitor and the ICI may be the same or different, and may be adjusted by those skilled in the art as needed to provide optimal therapeutic efficacy.

The present disclosure provides a use of an ICI in treating and/or preventing a tumor in a mammal with RIP2 gene deficiency.

In the use, the tumor may be a tumor related to RIP2 signaling pathway, preferably colorectal cancer, melanoma, lung cancer, breast cancer, liver cancer, gastric cancer, or pancreatic cancer.

The present disclosure provides a use of an ICI in the manufacture of a medicament for treating and/or preventing a tumor in a mammal with RIP2 gene deficiency.

In the use, the tumor may be a tumor related to RIP2 signaling pathway, preferably colorectal cancer, melanoma, lung cancer, breast cancer, liver cancer, gastric cancer, or pancreatic cancer.

In the use, the ICI may be one or more of a programmed death 1 (PD-1) inhibitor, a programmed death-ligand 1 (PD-L1) inhibitor, a cytotoxic T-lymphocyte-associated protein 4 (CTLA-4) inhibitor, a B- and T-lymphocyte attenuator (BTLA) inhibitor, a T cell immunoglobulin and mucin domain 3 (TIM-3) inhibitor, a T cell immunoreceptor with Ig and ITIM domains (TIGIT) inhibitor, a V-domain Ig suppressor of T cell activation (VISTA) inhibitor, a lymphocyte-activation gene 3 (LAG-3) inhibitor, and an indoleamine 2,3-dioxygenase 1 (IDO1) inhibitor; preferably, the ICI comprises a PD-1 inhibitor or a PD-L1 inhibitor.

In the use, the programmed death 1 (PD-1) inhibitor may be pembrolizumab.

In the use, the programmed death-ligand 1 (PD-L1) inhibitor may be atezolizumab.

In the use, the ICI may be administered at a dose that is conventional in the art, typically at a dose of 2 mg/kg to 10 mg/kg (per single dose), such as 6 mg/kg. For another example, the PD-L1 inhibitor (*e.g.,* atezolizumab) is administered at a dose of 6 mg/kg (per single dose).

Preferably, the ICI is administered at equal or unequal intervals during treatment.

In a certain embodiment, the ICI is administered at unequal intervals, once every 4 days or once every 7 days.

In a certain embodiment, the ICI is administered at unequal intervals, once every 4 days or once every 7 days, for a total of 4 doses, for example, on days 0, 4, 11, and 18.

In a certain embodiment, the ICI is administered via injection (*e.g.,* intravenous, subcutaneous, intraperitoneal, or intramuscular injection).

### Explanation of Terms:

Unless otherwise specified, the following terms used in the present disclosure shall have the meanings set forth below:
As used herein, the term "treatment" refers to therapeutic therapy. In the context of a specific disorder, treatment refers to: (1) ameliorating one or more biological manifestations of the disease or disorder; (2) interfering with (a) one or more points in the biological cascade that leads to or causes the disorder or (b) one or more biological manifestations of the disorder; (3) ameliorating one or more symptoms, effects, or side effects associated with the disorder, or one or more symptoms, effects, or side effects associated with the disorder or its treatment; or (4) slowing the progression of the disorder or one or more biological manifestations of the disorder.

As used herein, the term "therapeutically effective amount" refers to an amount of the pharmaceutical composition that, when administered to a patient, is sufficient to effectively treat the disease or disorder described herein. The amount of the pharmaceutical composition constituting a "therapeutically effective amount" will vary depending on the pharmaceutical composition, the disease and its severity, and the age of the patient to be treated, but may be adjusted as needed by those skilled in the art.

As used herein, the term "pharmaceutical composition" refers to a composition containing a specified active ingredient that can be formulated into the same dosage form.

As used herein, the term "patient" refers to any animal, preferably a mammal, most preferably a human, that is about to receive or has received the administration of the compound or composition according to an example of the present disclosure. As used herein, the term "mammal" includes any mammal. Examples of mammals include, but are not limited to, cattle, horses, sheep, pigs, cats, dogs, mice, rats, rabbits, guinea pigs, monkeys, humans, *etc.,* most preferably humans.

As used herein, the term "pharmaceutically acceptable excipient" refers to excipients and additives used in the manufacture of pharmaceutical products and the compounding of prescriptions, which are all substances contained in pharmaceutical preparations other than active ingredients. For reference, see Pharmacopoeia of the People's Republic of China (2020 Edition), Volume IV, or Handbook of Pharmaceutical Excipients (Raymond C Rowe, 2009 Sixth Edition).

On the basis of not violating common knowledge in the art, the preferred conditions described above may be combined arbitrarily to obtain preferred examples of the present disclosure.

The reagents and raw materials used in the present disclosure are all commercially available.

The positive and progressive effect of the present disclosure is that the pharmaceutical compositions of the present disclosure can enhance the anti-tumor activity of ICI medicaments by up-regulating the proportions of CD3+ cells, CD8+ T cells, γδTCR+ T cells, effector memory T cells, and CD8 effector T cells (IFNγ+ CD8+ T cells, Granzyme B+ CD8+ T cells, TNFα+ CD8+ T cells, and CD107a+ CD8+ T cells) in a tumor microenvironment, thereby enhancing the therapeutic effect of ICI medicaments on tumors.

### BRIEF DESCRIPTION OF THE DRAWINGS

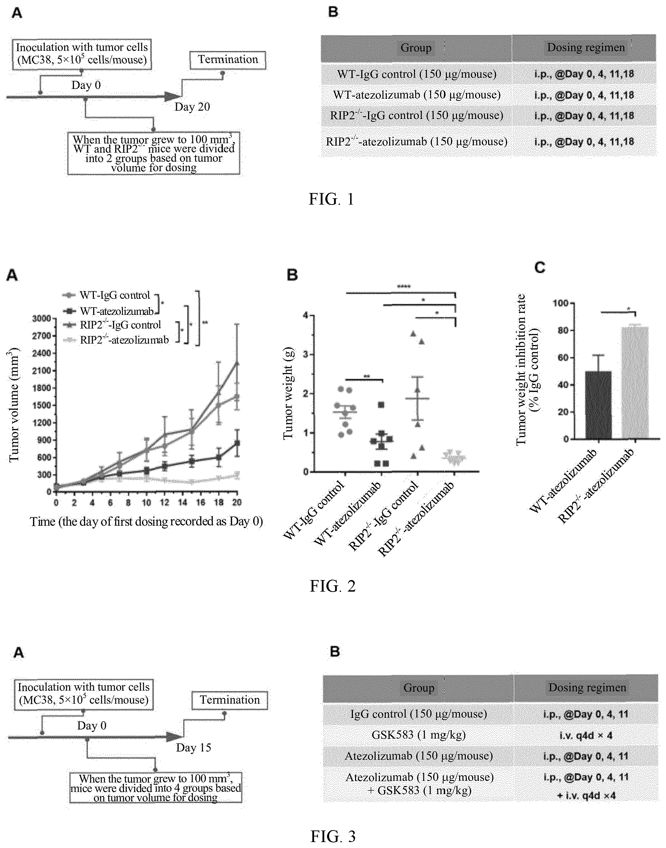

FIG. 1 shows the establishment of a colorectal cancer (MC38) tumor-bearing model in wild-type (WT) and RIP2 knockout (RIP2^{-/-}) C57BL/6 mice and the dosing regimen of the PD-L1 inhibitor (atezolizumab). A of FIG. 1 shows the establishment scheme of the colorectal cancer tumor-bearing model in mice. B of FIG. 1 shows the dosing regimen of atezolizumab for the treatment of the colorectal cancer tumor-bearing model in WT and RIP2^{-/-} C57BL/6 mice.
FIG. 2 shows that RIP2 knockout significantly enhances the inhibitory effect of the PD-L1 inhibitor (atezolizumab) on *in vivo* growth of colorectal cancer in mice. A of FIG. 2 shows a graph of changes in tumor volume growth across different treatment groups. B of FIG. 2 shows the results of tumor tissue weight across different treatment groups. C of FIG. 2 shows the results of tumor weight inhibition rate across different treatment groups.
FIG. 3 shows the establishment of a colorectal cancer (MC38) tumor-bearing model in C57BL/6 mice and the dosing regimen of the RIP2 inhibitor GSK583 combined with the PD-L1 inhibitor (atezolizumab). A of FIG. 3 shows the establishment scheme of the colorectal cancer tumor-bearing model in mice. B of FIG. 3 shows the dosing regimen of GSK583 combined with atezolizumab for the treatment of the colorectal cancer tumor-bearing model in mice.

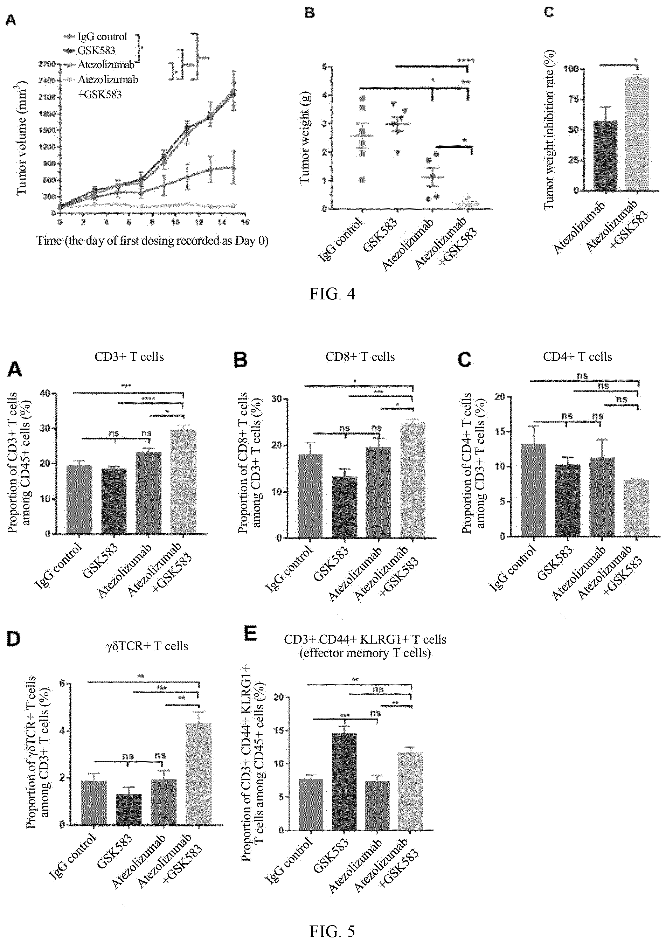

FIG. 4 shows that GSK583 significantly enhances the inhibitory effect of the PD-L1 inhibitor (atezolizumab) on *in vivo* growth of colorectal cancer in mice. A of FIG. 4 shows a graph of changes in tumor volume growth across different treatment groups. B of FIG. 4 shows the results of tumor tissue weight across different treatment groups. C of FIG. 4 shows the results of tumor weight inhibition rate across different treatment groups.
FIG. 5 shows that GSK583 combined with the PD-L1 inhibitor (atezolizumab) can increase the proportion of various immune cell types in tumor tissues of a colorectal cancer tumor-bearing model. A of FIG. 5 shows the proportion of CD3+ cells among immune cells in tumor tissues across different treatment groups. B of FIG. 5 shows the proportion of CD8+ cells among T cells in tumor tissues across different treatment groups. C of FIG. 5 shows the proportion of CD4+ cells among T cells in tumor tissues across different treatment groups. D of FIG. 5 shows the proportion of γδTCR+ T cells among T cells in tumor tissues across different treatment groups. E of FIG. 5 shows the proportion of effector memory T cells among immune cells.

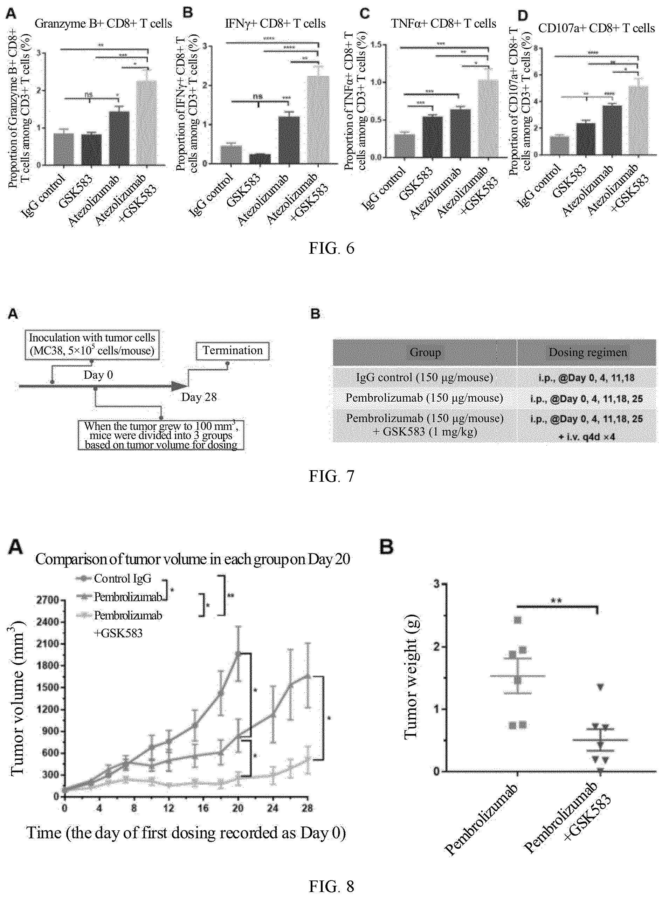

FIG. 6 shows that GSK583 combined with the PD-L1 inhibitor (atezolizumab) can increase the proportion of CD8 effector T cells in tumor tissues of a colorectal cancer tumor-bearing model. A of FIG. 6 shows the proportion of Granzyme B+ CD8+ T cells among T cells in tumor tissues across different treatment groups. B of FIG. 6 shows the proportion of IFNγ+ CD8+ T cells among T cells in tumor tissues across different treatment groups. C of FIG. 6 shows the proportion of TNFα+ CD8+ T cells among T cells in tumor tissues across different treatment groups. D of FIG. 6 shows the proportion of CD107a+ CD8+ T cells among T cells in tumor tissues across different treatment groups.
FIG. 7 shows the establishment of a colorectal cancer (MC38) tumor-bearing model in C57BL/6 mice and the dosing regimen of the RIP2 inhibitor GSK583 combined with the PD-1 inhibitor (pembrolizumab). A of FIG. 7 shows the establishment scheme of the colorectal cancer tumor-bearing model in mice. B of FIG. 7 shows the dosing regimen of GSK583 combined with pembrolizumab for the treatment of the colorectal cancer tumor-bearing model in mice.
FIG. 8 shows that GSK583 significantly enhances the inhibitory effect of the PD-1 inhibitor (pembrolizumab) on *in vivo* growth of colorectal cancer in mice. A of FIG. 8 shows a graph of changes in tumor volume growth across different treatment groups. B of FIG. 8 shows the results of tumor tissue weight across different treatment groups.

### DETAILED DESCRIPTION OF THE PREFERRED EMBODIMENT

The examples of the present disclosure are described in detail below. The examples described below are exemplary and are only used to explain the present disclosure, and cannot be construed as limiting the present disclosure. The examples without indication of specific techniques or conditions shall be implemented in accordance with the techniques or conditions described in the literature in the art or in accordance with the product specification. The reagents or instruments used without indication of the manufacturer are all commercially available conventional products.

Unless otherwise specified, "*" in the accompanying drawings of the present disclosure indicates significant difference, wherein "*" indicates p<0.05, "**" indicates p<0.01, "***" indicates p<0.001, "****" indicates p<0.0001, and "ns" indicates p>0.05, which means no significant difference.

### Example 1: Significant enhancement of inhibition of PD-L1 inhibitor (atezolizumab) on in vivo growth of colorectal cancer (MC38) in C57BL/6 mice by RIP2 knockout

Test substances atezolizumab (Roche) and IgG control antibody (inVivoMAb human IgG1 isotype control; BioXCell, BE0297) were prepared in saline. Experimental animals and tumor lines: SPF-grade male wild-type (WT) C57BL/6 mice (20 to 25 g) and RIP2 knockout (RIP2^{-/-}) mice (6 to 8 weeks old, 20 to 25 g) were purchased from Shanghai Model Organisms Center, Inc. The mice were generated using CRISPR-Cas9 technology and bred at the Laboratory Animal Resources Center, Tsinghua University. The cells used in the experiment were murine colorectal cancer cells (MC38), which were purchased from the Cell Bank of Peking Union Medical College.

According to the method shown in (A and B of FIG. 1), the mice were subcutaneously inoculated with MC38 cells at a concentration of 5 × 10⁶ cells/mL and 0.1 mL/mouse. When the tumor grew to a volume within the range of 100 mm³, WT mice and RIP2^{-/-} mice were randomly divided into 2 groups based on tumor volume, with 6 to 8 mice per group, and dosing was initiated. The dosing regimen was as follows: (i) WT-blank control (WT-IgG control, 150 µg/mouse) group; (ii) WT-atezolizumab (150 µg/mouse) group; (iii) RIP2^{-/-}-blank control (RIP2^{-/-}-IgG control, 150 µg/mouse) group; (iv) RIP2^{-/-}-atezolizumab (150 µg/mouse) group. IgG control and atezolizumab were administered via intraperitoneal injection on Days 0, 4, 11, and 18 for a total of 4 doses. The day of grouping and dosing was recorded as Day 0. During the dosing process, the animals were weighed every 2 to 3 days, and the long and short diameters of the tumor were measured with a vernier caliper. The tumor size was calculated by the formula: (1/2) × long diameter × (short diameter)². The experiment was terminated on Day 20. After the mice were euthanized and dissected, the tumor was removed and weighed, and the tumor inhibition rate was calculated by the formula: [(tumor weight of the treatment group - average tumor weight of the control group) / average tumor weight of the control group] × 100%.

### Experimental results (as shown in FIG. 2):

In the colorectal cancer (MC38) tumor-bearing model in WT and RIP2^{-/-} C57BL/6 mice, compared to the corresponding IgG control group, atezolizumab significantly inhibited tumor growth, with slower tumor volume progression, and the inhibitory effect was more pronounced in the RIP2^{-/-}-atezolizumab group; compared to WT mice, the inhibitory effect of atezolizumab on tumor growth was also significantly improved in RIP2^{-/-} mice (A of FIG. 2). At the end of dosing, compared to the corresponding control group, atezolizumab significantly reduced tumor weight in both WT and RIP2^{-/-} mice, with statistical significance (B of FIG. 2); the anti-tumor effect of atezolizumab in RIP2^{-/-} mice was significantly stronger than that in WT mice (B and C of FIG. 2). The results demonstrate that RIP2 knockout significantly enhanced the inhibitory effect of the PD-L1 inhibitor on *in vivo* growth of colorectal cancer in mice.

### Example 2: Significant enhancement of inhibition of PD-L1 inhibitor (atezolizumab) on in vivo growth of colorectal cancer (MC38) in C57BL/6 mice by RIP2 inhibitor GSK583

Test substances atezolizumab (Roche) and IgG control antibody (inVivoMAb human IgG1 isotype control; BioXCell, BE0297) were prepared in saline. GSK583 was formulated as a 20× stock solution in DMSO and diluted to the dosing concentration with saline containing 5% polyoxyl 35 castor oil (Cremophor EL) before use.

Experimental animals and tumor lines: SPF-grade male wild-type C57BL/6 mice (6 to 8 weeks old, 20 to 25 g) were purchased from the Laboratory Animal Resources Center, Tsinghua University. The cells used in the experiment were murine colorectal cancer cells (MC38), which were purchased from the Cell Bank of Peking Union Medical College.

According to the method shown in (A and B of FIG. 3), the mice were subcutaneously inoculated with MC38 cells at a concentration of 5 × 10⁶ cells/mL and 0.1 mL/mouse. When the tumor grew to a volume within the range of 100 mm³, the mice were randomly divided into 4 groups based on tumor volume, with 6 to 8 mice per group, and dosing was initiated. The dosing regimen was as follows: (i) blank control (IgG control, 150 µg/mouse) group; (ii) GSK583 (1 mg/kg) group; (iii) atezolizumab (150 µg/mouse) group; (iv) atezolizumab (150 µg/mouse) + GSK583 (1 mg/kg). GSK583 was administered via intravenous injection once every 4 days for a total of 4 doses; IgG control and atezolizumab were administered via intraperitoneal injection on Days 0, 4, and 11 for a total of 3 doses. The day of grouping and dosing was recorded as Day 0. During the dosing process, the animals were weighed every 2 to 3 days, and the long and short diameters of the tumor were measured with a vernier caliper. The tumor size was calculated by the formula: (1/2) × long diameter × (short diameter)². The experiment was terminated on Day 15. After the mice were euthanized and dissected, the tumor was removed and weighed, and the tumor inhibition rate was calculated.

### Experimental results (as shown in FIG. 4):

In the colorectal cancer (MC38) tumor-bearing model in C57BL/6 mice, compared to the IgG control group, both the atezolizumab group and the atezolizumab + GSK583 combination group significantly inhibited tumor growth, with slower tumor volume progression (A of FIG. 4). At the end of dosing, the tumor weight of animals in both groups was significantly lower than that in the control group, with statistical significance (B and C of FIG. 4); the anti-tumor effect of atezolizumab + GSK583 was significantly stronger than that of atezolizumab alone (B and C of FIG. 4). GSK583 alone had no significant inhibitory effect on tumor growth (A, B, and C of FIG. 4). The results demonstrate that the RIP2 inhibitor GSK583 significantly enhanced the inhibitory effect of the PD-L1 inhibitor on *in vivo* growth of colorectal cancer in mice.

### Example 3: Flow cytometry detection of proportion of immune cells in tumor tissues of colorectal cancer-bearing mice treated with GSK583 combined with PD-L1 inhibitor (atezolizumab)

Preparation of enzyme lysis buffer: 1 g of collagenase IV (Sigma, Cat: C5138), 100 mg of hyaluronidase (Sigma, Cat: H6254), and 20,000 units of DNase type IV (Sigma, Cat: D5025) were added to 80 mL of Hank's Balanced Salt Solution (HBSS; EallBio Life Science, Cat: 03.15009C) and mixed well. The mixture was then added with HBSS to make up a volume of 100 mL, yielding a 100× tissue lysis buffer, which was filtered through a 0.22 µm filter membrane and stored at -20°C, and diluted to 1× with HBSS before use.

Dissociation of tumor tissues: The tumor tissues obtained from each group in Example 2 were removed and weighed (0.04 to 1 g), then cut into small pieces of 2 × 2 mm with a scalpel, added with 2.5 mL of lysis mixture, and transferred to a gentleMACS C Tube (Miltenyi Biotec; Cat: 130-096-334). The C Tube was inverted on a gentleMACS Dissociator (Miltenyi Biotec; Cat: 130-093-235), and the program "gentleMACS Program m_impTumor_02" was run. The C Tube was removed from the gentleMACS Dissociator and incubated at 37°C for 40 minutes. The C Tube was inverted on the gentleMACS Dissociator again, and the program "gentleMACS Program m_impTumor_03" was run.

The mixture was filtered through a 70 µm filter membrane (BD Biosciences, Cat: 352235), centrifuged at 300 × g for 7 minutes, and the supernatant was discarded. 5 mL of red blood cell lysis buffer (BioLegend, Cat: 420301) was added, then the mixture was lysed at room temperature for 5 minutes, and 10 mL of phosphate-buffered saline (PBS; EallBio Life Science, Cat: 03.15018C) was added to terminate the reaction. The mixture was centrifuged at 1000 rpm for 5 minutes at 4°C, and the supernatant was discarded. Cell staining buffer (BioLegend, Cat: 420201) was added and mixed well.

Flow cytometry detection: After counting, the dissociated tumor cells were adjusted to an appropriate concentration, and 200 µL/well of cells (5 to 10 × 10⁵ cells/well) were added to a 96 U-bottom plate. The plate was centrifuged at 350 × g for 5 minutes, and the supernatant was discarded. 100 µL/well of blocking buffer containing CD16/CD32 (BioLegend; Cat: 101302, diluted 1:200 in cell staining buffer) was added, and the cells were incubated in the dark at 4°C for 15 to 20 minutes. The plate was then centrifuged at 350 × g for 5 minutes, and the supernatant was discarded. 100 µL/well of pre-mixed antibody cocktail was added, and the cells were incubated in the dark at 4°C for 30 minutes. The plate was centrifuged at 350 × g for 5 minutes, and the supernatant was discarded. 100 µL/well of cell staining buffer was then added for washing. The plate was centrifuged at 350 × g for 5 minutes, and the supernatant was discarded. The washing was repeated twice. After the last washing, the cells were resuspended in 100 µL/well of 4% paraformaldehyde (Beyotime, P0099) and incubated overnight at 4°C. The plate was centrifuged at 350 × g for 5 minutes, and the supernatant was discarded. The cells were resuspended in 100 µL/well of cell staining buffer, and then analyzed by flow cytometry.

### Experimental results (as shown in FIG. 5 or FIG. 6):

Compared to the IgG control group, the GSK583 group, and the atezolizumab group, the atezolizumab + GSK583 combination group showed a significant increase in the proportions of CD3+ cells, CD8+ T cells, γδTCR+ T cells, and effector memory T cells in tumor tissues, while the proportion of CD4+ T cells remained unaffected (A to E of FIG. 5) Further analysis on different types of CD8 effector T cells revealed that the atezolizumab + GSK583 combination group showed a significant increase in the proportions of IFNγ+ CD8+ T cells, Granzyme B+ CD8+ T cells, TNFα+ CD8+ T cells, and Cd107a+ CD8+ T cells among CD8 effector T cells (A to D of FIG. 6). Therefore, it can be judged that the combined use of the RIP2 inhibitor and the ICI up-regulates the proportions of CD3+ cells, CD8+ T cells, γδTCR+ T cells, and effector memory T cells in a tumor microenvironment, and increases the proportions of IFNγ+ CD8+ T cells, Granzyme B+ CD8+ T cells, TNFα+ CD8+ T cells, and Cd107a+ CD8+ T cells among CD8 effector T cells, thereby enhancing the inhibitory effect of ICI medicaments on tumor growth.

### Example 4: Significant enhancement of inhibition of PD-1 inhibitor (pembrolizumab) on in vivo growth of colorectal cancer (MC38) in C57BL/6 mice by RIP2 inhibitor GSK583

Test substances pembrolizumab (Merck & Co., Inc.) and IgG control antibody (inVivoMAb human IgG1 isotype control; BioXCell, BE0297) were prepared in saline. GSK583 was formulated as a 20× stock solution in DMSO and diluted to the dosing concentration with saline containing 5% polyoxyl 35 castor oil (Cremophor EL) before use.

Experimental animals and tumor lines: SPF-grade male wild-type C57BL/6 mice (6 to 8 weeks old, 20 to 25 g) were purchased from the Laboratory Animal Resources Center, Tsinghua University. The cells used in the experiment were murine colorectal cancer cells (MC38), which were purchased from the Cell Bank of Peking Union Medical College.

According to the method shown in (A and B of FIG. 7), the mice were subcutaneously inoculated with MC38 cells at a concentration of 5 × 10⁶ cells/mL and 0.1 mL/mouse. When the tumor grew to a volume within the range of 100 mm³, the mice were randomly divided into 3 groups based on tumor volume, with 6 to 7 mice per group, and dosing was initiated. The dosing regimen was as follows: (i) blank control (IgG control, 150 µg/mouse) group; (ii) pembrolizumab (150 µg/mouse) group; (iii) pembrolizumab (150 µg/mouse) + GSK583 (1 mg/kg) group. GSK583 was administered via intravenous injection once every 4 days for a total of 7 doses; IgG control was administered via intraperitoneal injection on Days 0, 4, 11, and 18 for a total of 4 doses; pembrolizumab was administered via intraperitoneal injection on Days 0, 4, 11, 18, and 25 for a total of 5 doses. The day of grouping and dosing was recorded as Day 0. During the dosing process, the animals were weighed every 2 to 3 days, and the long and short diameters of the tumor were measured with a vernier caliper. The tumor size was calculated by the formula: (1/2) × long diameter × (short diameter)². The average tumor volume of mice in the IgG control group was close to 2000 mm³ on Day 20, at which point the mice in this group were euthanized. The experiment was terminated on Day 28, at which point the mice in the pembrolizumab group and the pembrolizumab (150 µg/mouse) + GSK583 (1 mg/kg) group were euthanized and dissected, and the tumor was removed and weighed.

### Experimental results (as shown in FIG. 8):

In the colorectal cancer (MC38) tumor-bearing model in C57BL/6 mice, compared to the IgG control group, both pembrolizumab and pembrolizumab + GSK583 significantly inhibited tumor growth on Day 20, with slower tumor volume progression (A of FIG. 8). At the end of dosing on Day 28, the tumor weight of animals in pembrolizumab + GSK583 group was significantly lower than that in the pembrolizumab group, with statistical significance (B of FIG. 8). The results demonstrate that the RIP2 inhibitor GSK583 significantly enhanced the inhibitory effect of the PD-1 inhibitor on *in vivo* growth of colorectal cancer in mice.

Although the specific embodiments of the present disclosure have been described above, those skilled in the art should understand that these are merely illustrative examples. Various changes or modifications can be made to these embodiments without departing from the principles and essence of the present disclosure. Therefore, the scope of protection for the present disclosure is defined by the appended claims.

## Claims

1. A pharmaceutical composition of an RIP2 inhibitor in combination with an ICI, comprising an RIP2 inhibitor and an ICI.

2. The pharmaceutical composition according to claim 1, wherein the composition satisfies one or more of the following conditions:
(1) the pharmaceutical composition further comprises a pharmaceutically acceptable excipient;
(2) the ICI is one or more of a programmed death 1 inhibitor, a programmed death-ligand 1 inhibitor, a cytotoxic T-lymphocyte-associated protein 4 inhibitor, a B- and T-lymphocyte attenuator inhibitor, a T cell immunoglobulin and mucin domain 3 inhibitor, a T cell immunoreceptor with Ig and ITIM domains inhibitor, a V-domain Ig suppressor of T cell activation inhibitor, a lymphocyte-activation gene 3 inhibitor, and an indoleamine 2,3-dioxygenase 1 inhibitor; preferably, the ICI comprises a PD-L1 inhibitor and/or a PD-1 inhibitor, wherein the PD-L1 inhibitor may be atezolizumab, and the PD-1 inhibitor may be pembrolizumab;
(3) the mass ratio of the RIP2 inhibitor to the ICI is 1:(1 to 60), such as 1:6, 1:10, 1:15, 1:20, 1:30, or 1:60, preferably 1:(6 to 10);
and (4) the RIP2 inhibitor is one or more of GSK583, WEHI-345, SB203580, OD36, OD38, and a derivative thereof, preferably GSK583 and/or a derivative thereof; the derivative of GSK583 is preferably GSK2983559.

3. The pharmaceutical composition according to claim 1 or 2, wherein the pharmaceutical composition consists of the RIP2 inhibitor and the ICI;
preferably, the pharmaceutical composition is GSK583 and a PD-1 inhibitor, such as GSK583 and pembrolizumab, and the mass ratio of the GSK583 to the PD-1 inhibitor is further preferably 1:6 to 1:10; or, the pharmaceutical composition is GSK583 and a PD-L1 inhibitor, such as GSK583 and atezolizumab, and the mass ratio of the GSK583 to the PD-L1 inhibitor is further preferably 1:6 to 1:10.

4. The pharmaceutical composition according to at least one of claims 1 to 3, wherein the pharmaceutical composition satisfies one or more of the following conditions:
(1) the RIP2 inhibitor is administered at a dose of 0.1 mg/kg to 5 mg/kg, such as 1 mg/kg;
(2) the ICI is administered at a dose of 2 to 10 mg/kg, such as 6 mg/kg;
(3) the RIP2 inhibitor is administered once every 4 days;
(4) the ICI is administered once every 4 days or once every 7 days;
and (5) the RIP2 inhibitor is administered at equal intervals during treatment, and the ICI is administered at equal or unequal intervals during treatment.

5. The pharmaceutical composition according to at least one of claims 1 to 4, wherein the pharmaceutical composition satisfies one or more of the following conditions:
(1) the RIP2 inhibitor is administered once every 4 days, for a total of 4 to 7 doses, for example, on days 0, 4, 8, and 12;
(2) the ICI is administered at unequal intervals, once every 4 days or once every 7 days, for a total of 3, 4, or 5 doses, for example, on days 0, 4, 11, and 18;
and (3) both the RIP2 inhibitor and the ICI are administered via injection, such as intravenous, subcutaneous, intramuscular, or intraperitoneal injection.

6. Use of the pharmaceutical composition according to at least one of claims 1 to 5 in the manufacture of a medicament for treating and/or preventing a tumor.

7. The use according to claim 6, wherein the tumor is a tumor related to RIP2 signaling pathway, preferably colorectal cancer, melanoma, lung cancer, breast cancer, liver cancer, gastric cancer, or pancreatic cancer.

8. A method for treating and/or preventing a tumor, comprising administering a therapeutically effective amount of the pharmaceutical composition according to at least one of claims 1 to 5 to a patient in need thereof.

9. The method according to claim 8, wherein the RIP2 inhibitor is administered at a dose of 0.1 mg/kg to 5 mg/kg, for example, 1 mg/kg of GSK583 is administered; or, the ICI is administered at a dose of 2 mg/kg to 10 mg/kg, for example, 6 mg/kg of atezolizumab or pembrolizumab is administered.

10. The method according to claim 8 or 9, wherein the method satisfies one or more of the following conditions:
(1) the RIP2 inhibitor is administered at a dose of 1 mg/kg;
(2) the ICI is administered at a dose of 6 mg/kg;
(3) the RIP2 inhibitor is administered once every 4 days, for a total of 4 to 7 doses, for example, on days 0, 4, 8, and 12;
(4) the ICI is administered at unequal intervals, once every 4 days or once every 7 days, for a total of 3, 4, or 5 doses, for example, on days 0, 4, 11, and 18;
(5) both the RIP2 inhibitor and the ICI are administered via injection, such as intravenous, subcutaneous, intramuscular, or intraperitoneal injection;
and (6) the tumor is a tumor related to RIP2 signaling pathway, preferably melanoma, lung cancer, breast cancer, colorectal cancer, gastric cancer, or pancreatic cancer.

11. Use of an ICI in the manufacture of a medicament for treating and/or preventing a tumor in a mammal with RIP2 gene deficiency.

12. The use according to claim 11, wherein the use satisfies one or more of the following conditions:
(1) the tumor is a tumor related to RIP2 signaling pathway, preferably colorectal cancer, melanoma, lung cancer, breast cancer, liver cancer, gastric cancer, or pancreatic cancer;
(2) the ICI is one or more of a programmed death 1 inhibitor, a programmed death-ligand 1 inhibitor, a cytotoxic T-lymphocyte-associated protein 4 inhibitor, a B- and T-lymphocyte attenuator inhibitor, a T cell immunoglobulin and mucin domain 3 inhibitor, a T cell immunoreceptor with Ig and ITIM domains inhibitor, a V-domain Ig suppressor of T cell activation inhibitor, a lymphocyte-activation gene 3 inhibitor, and an indoleamine 2,3-dioxygenase 1 inhibitor; preferably, the ICI comprises a PD-L1 inhibitor and/or a PD-1 inhibitor, wherein the PD-L1 inhibitor may be atezolizumab, and the PD-1 inhibitor may be pembrolizumab;
(3) the ICI is administered at a dose of 2 to 10 mg/kg, such as 6 mg/kg;
(4) the ICI is administered at unequal intervals, once every 4 days or once every 7 days;
and (5) the ICI is administered via injection, such as intravenous, subcutaneous, intraperitoneal, or intramuscular injection.
